# EUROPÄISCHE PATENTSCHRIFT

(11) **EP 1 522 529 B1**
(45) Veröffentlichungstag und Bekanntmachung des Hinweises auf die Patenterteilung: **07.01.2009**
(21) Anmeldenummer: 04023834.7
(22) Anmeldetag: 06.10.2004
(51) Int. Cl.: C02F 9/00, C02F 11/04, C02F 11/10

(54) **Vorrichtung zum Verwerten von Flüssigabfällen**
Device for using liquid waste
Dispositif pour traiter des déchets liquides

(30) Priorität: 07.10.2003 DE 20315555 U
(43) Veröffentlichungstag der Anmeldung: 13.04.2005
(73) Patentinhaber: Inora AG, 6304 Zug (CH)
(72) Erfinder: Lafontaine, Hans Jörg, 66787 Wadgassen (DE); Hildesheim, Klaus-Thomas, 66578 Schiffweiler (DE)
(74) Vertreter: Vièl, Christof

(56) Entgegenhaltungen:
- WO-A-01/53510
- DE-A1- 2 604 470
- DE-A1- 3 330 542
- FR-A- 2 787 103
- GB-A- 2 233 340
- US-A- 4 289 625
- US-A- 4 321 151
- PATENT ABSTRACTS OF JAPAN Bd. 2003, Nr. 12, 5. Dezember 2003 (2003-12-05) & JP 2003 290750 A (TAKUMA CO LTD), 14. Oktober 2003 (2003-10-14)
- PATENT ABSTRACTS OF JAPAN Bd. 2002, Nr. 10, 10. Oktober 2002 (2002-10-10) -& JP 2002 159995 A (KURITA WATER IND LTD), 4. Juni 2002 (2002-06-04)
- DATABASE WPI Section Ch, Week 199821 Derwent Publications Ltd., London, GB; Class D15, AN 1998-238199 XP002316911 -& RU 2 091 329 C1 (KHABAROVSK METAL CONSTRUCTIONS WKS) 27. September 1997 (1997-09-27)

## Beschreibung

Die Erfindung betrifft eine Vorrichtung zum Verwerten von Flüssigabfällen.

Unter Flüssigabfällen versteht man beispielsweise fermentierbare Flüssigabfälle, wie nicht mehr zum Verzehr geeignete Lebensmittel, Produktionsabfälle, Fettabscheiderinhalte, flüssige Industrieklärschlämme oder nicht fermentierbare Flüssigabfälle, wie z.B. ÖI-Wasser-Gemische beziehungsweise Mischungen hiervon.

Vorrichtungen zum Verwerten von Flüssigabfällen sind zwar bekannt, diese haben jedoch in der Regel gravierende Nachteile. So ist in aller Regel aufgrund einer unzureichenden Nutzung der in den Flüssigabfällen enthaltenen Energie die energetische Bilanz derartiger Vorrichtungen negativ; es muß also der Vorrichtung Energie zugeführt werden, was einer breiten Akzeptanz zuwiderläuft. Zudem sind die meisten derartigen Vorrichtungen auf die Verwertung einer Kategorie der obengenannten Flüssigabfälle abgestimmt und Umstellungen hinsichtlich der Einsatzstoffe sind problematisch.

So ist aus der DE 100 21 383 A1 und der DE 43 02 740 A1 eine Vorrichtung bekannt, die für die Annahme ausschließlich fester Abfallstoffe vorgesehen ist, wobei diese keinesfalls besonders überwachungsbedürftig gemäß europäischem Abfallrecht sein dürfen, da die überschüssige Schlammfraktion auf Felder ausgebracht wird. Eine Bearbeitung von flüssigen bzw. Feststoff enthaltenden Flüssigkeiten, die auch besonders überwachungsbedürftig sein können, ist nicht möglich.

Die DE 43 02 740 A1 beschreibt eine Schwelgasanlage, bei der der Verschwelungsprozeß mit Sauerstoffunterschuß betrieben und die zur Verschwelung notwendige thermische Energie durch Teilverbrennung des zu verschwelenden Materials erzeugt wird. Die Abfälle, welche die hier beschriebene Technologie verarbeiten kann, dürfen keinesfalls besonders überwachungsbedürftig gemäß europäische Abfallrecht sein (die überschüssige Schlammfraktion wird auf Felder ausgebracht).

Die in der DE 101 07 712 A1 beschriebene Technologie dient ausschließlich zur Behandlung von kommunalem Klärschlamm aus einer Belebungsstufe. Die Technologie ist angewiesen auf vorhandene Faultürme einer kommunalen Kläranlage (siehe Ansprüche). In diesem Fall wird der Faulturm dazu benutzt, das Mischgas energiereicher zu machen.

Aus der DE 41 15 435 C1 ist eine Technologie bekannt, die ausschließlich zur Behandlung von Explosivstoffen dient. Das Verfahren wird in Kombination oder parallel zu vorhandenen Kläranlagen betrieben und stellt somit keine eigenständige Lösung dar.

In der DE 101 07 712 A1 wird im wesentlichen ein Trockenfermenter beschrieben. Es wird ausschließlich Biomüll verarbeitet, also Feststoffe. Die Verarbeitung von Flüssigkeiten bzw. besonders überwachungsbedürftigen Abfällen gemäß EU-Abfallrecht schließt dieses Verfahren aus.

Bei dem Verfahren gemäß der WO 01/53510 A1 wird in einem ersten Schritt aerob fermentiert, d.h. kompostiert, also unter Luftzufuhr einen Stoffumsatz erreicht. Im zweiten Schritt wird das organische Material verschwelt. Bei einer Schwelgasanlage wird der Verschwelungsprozeß mit Sauerstoffunterschuß betrieben und die zur Verschwelung notwendige thermische Energie durch Teilverbrennung des zu verschwelenden Materials erzeugt wird. Im dritten Verfahrensschritt wird aus dem Schwelgas = Holzgas unter anaeroben thermophilen Bedingungen Methangas erzeugt.

Aufgabe dieser Erfindung ist es, eine Vorrichtung zum Verwerten von Flüssigabfällen zu schaffen, deren energetische Bilanz zumindest ausgeglichen, wenn nicht sogar positiv ist und die für eine breite Palette von Flüssigabfällen geeignet ist.

Diese Aufgabe wird erfindungsgemäß dadurch gelöst, daß die Vorrichtung eine Belebungsanlage und eine Fermentieranlage umfaßt, wobei eine Separiervorrichtung zum Trennen von fermentierbaren und nicht fermentierbaren Flüssigabfällen vorgesehen ist sowie Mittel zum Weiterleiten der fermentierbaren Flüssigabfälle zu der Fermentieranlage sowie Mittel zum Weiterleiten der nicht fermentierbaren Flüssigabfälle zu der Belebungsanlage vorgesehen sind, daß eine Sedimentationsstufe und eine Faulschlammentwässerungseinheit für den in der Fermentieranlage anfallenden Faulschlamm vorgesehen sind, daß sich an die Faulschlammentwässerungseinheit eine Faulschlammtrocknungseinheit anschließt und daß eine Pyrolyseeinheit zur Entgasung des getrockneten Faulschlamms aus der Faulschlammtrocknungseinheit vorgesehen ist.

Mit diesen Grundkomponenten können Flüssigabfälle verschiedenster Art verwertet werden, wobei die organischen Einsatzstoffe in Strom- und Wärmeenergie umgewandelt werden. Die Separieranlage dient dazu, nicht fermentierbare Flüssigabfälle an der Fermentieranlage vorbei direkt in die Belebungsanlage zu leiten. Die Sedimentationsstufe und die Faulschlammentwässerungseinheit sind Vorstufen zu der nachfolgenden Trocknung des Faulschlamms. In der Faulschlammentwässerungseinheit und der Faulschlammtrocknungseinheit wird der Faulschlamm getrocknet, wobei ein Trocknungsgrad von mehr als 90 %, vorzugsweise 95 %, angestrebt wird. In der Pyrolyseeinheit wird der getrocknete Faulschlamm entgast, wobei die Pyrolyseeinheit bevorzugt über eine Wirbelschichtfeuerung beheizt wird.

Eine Ausbildung der Erfindung besteht darin, daß die Belebungsanlage Mittel zum Abbau der Kohlenstoff-Fracht, Mittel zur Abwasserreinigung und kombinierten Denitrifikation umfaßt.

In der Belebungsanlage werden enthaltene organische Anteile in den Abfällen in Biomasse umgewandelt.

Vorteilhaft ist es, daß die Mittel zur Abwasserreinigung eine Pflanzenkläranlage umfassen.

In einer derartigen Pflanzenkläranlage kann das Abwasser auf Direkteinleiterqualität endgereinigt werden.

Weiterhin ist es zweckmäßig, daß die Fermentieranlage ein- oder mehrstraßig ausgebildet ist und einen oder mehrere mesophile und/oder thermophile Fermenter umfaßt.

In einer bevorzugten Variante sind die Fermenter zweistraßig angelegt und bestehen je Straße aus einem mesophil und einem thermophil arbeitenden Fermenter.

Vorzugsweise handelt es sich bei den Fermentern um Hochlastfermenter in Schlaufenreaktortechnologie.

Ebenfalls ist es erfindungsgemäß, daß Mittel zum Zuführen des Überschußschlamms aus der Belebungsanlage in die Fermentieranlage vorgesehen sind.

Der Überschußschlamm aus der Belebungsanlage sowie die direkt fermentierbaren Flüssigkeiten werden dann in der Fermentieranlage fermentiert.

Es ist vorteilhaft, daß die Faulschlammentwässerungseinheit als Zentrifuge ausgebildet ist.

Eine Zentrifuge ist für die Entwässerung von Faulschlamm geeignet, wobei auch andere Vorrichtungen (z.B. Pressen) grundsätzlich einsetzbar sind. Weiterhin ist es sinnvoll, daß Mittel zum Leiten des Pyrolysegases durch die Fermentierungsanlage vorgesehen sind.

Dieser Schritt ist von besonderem Vorteil, da hierdurch die sonst notwendige Pyrolysegasreinigung entfallen kann. Weiterer Vorteil ist der Ausschluß einer Geruchsbelastung der bei dem Trocknungsvorgang entstehenden sehr geruchsintensiven Brüden.

Schließlich liegt es im Rahmen der Erfindung, daß Mittel zum Leiten von Bio- und des Pyrolysegasen aus der Fermentierungsanlage zu einem Blockheizkraftwerk vorgesehen sind. Hierbei können Mittel zum Reinigen und Trocknen sowie Lagern der Gase verwendet werden.

Aufgrund der eingesetzten Flüssigabfälle besitzt das in der Fermentierungsanlage gewaschene Pyrolysegas einen ausreichenden Heizwert und bedarf keiner weiteren Anreicherung. In diesem Blockheizkraftwerk wird dann aus dem Bio- und dem Pyrolysegas Energie erzeugt, wobei die überschüssige elektrische Energie über eine Transformatorstation in das regionale Mittelspannungsnetz eingespeist werden kann. Überschüssige Wärmeenergie kann mittels eines Nahwärmenetzes an benachbarte Betriebe abgegeben werden.

Nachfolgend wird die Erfindung anhand eines Ausführungsbeispiels näher erläutert.

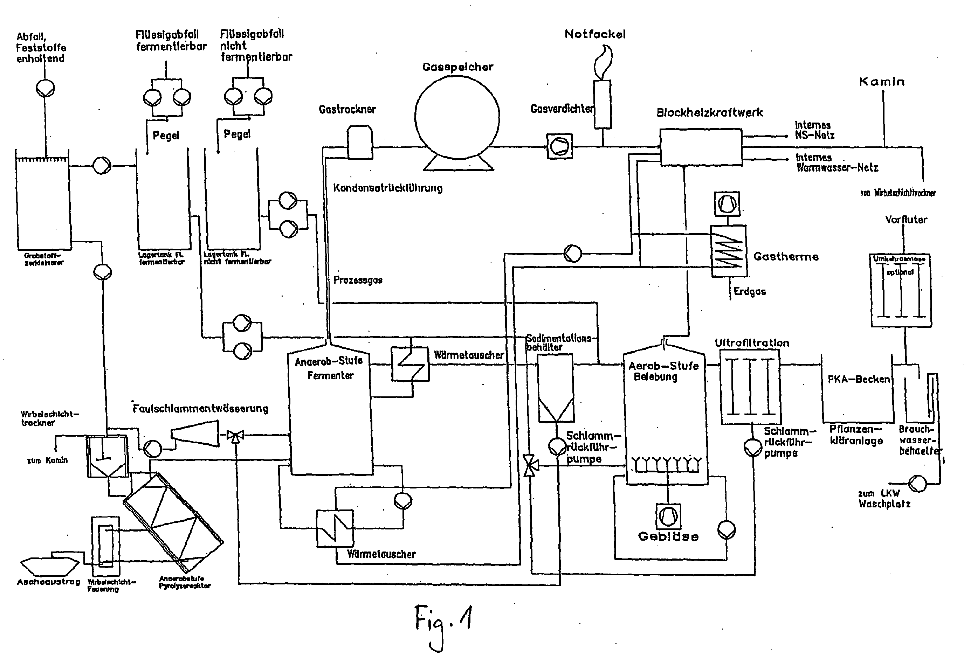

Es zeigt
- Fig. 1: eine schematische Darstellung (Fließbild) einer erfindungsgemäßen Vorrichtung.

Die Fahrzeuge mit den Flüssigabfällen werden vor der Entladung auf einer dafür vorgesehenen geeichten Bodenwaage verwogen. Die Anlieferung der Flüssigabfälle erfolgt durch Tanklastzüge. Diese werden an Andockstationen an der Maschinenhalle durch anlagenseitig installierte Pumpen entleert. Vor Freigabe der Entleerung werden die angelieferten besonders überwachungsbedürftigen Abfälle beprobt (Rückstellprobe und Gegenprobe). Nur gemäß Deklarationsanalytik organohalogenfreie Abfälle werden angenommen. Die Flüssigkeiten werden je nach Gruppe (fermentierbar oder nicht) in die Lagerbehälter gepumpt. Die hierfür notwendige Pumpanlage befindet sich in der Maschinenhalle.

Abfälle mit festen Bestandteilen werden entweder auf Paletten oder in Tonnen unterschiedlicher Volumina angeliefert. Diese werden über einen im Betrieb geschlossener Einwurftrichter einem in der flüssigen Phase arbeitenden Zerkleinerer zugeführt. Diesem ist eine Fest-flüssig-Trennung nachgeschaltet. Die flüssige Phase wird dem Lagerbehälter für fermentierbare Flüssigkeiten zugeführt, die Grobstoffe gelangen über eine geschlossene Transportschnecke in den Wirbelschichttrockner. Die Ladeflächen der anliefernden Fahrzeuge und die Erfassungsbehältnisse werden entsprechend den gesetzlichen Vorgaben auf einem dafür vorgesehenen Waschplatz gereinigt und desinfiziert.

Zur Lagerung und Vergleichmäßigung des Inputs der beiden biologischen Behandlungsstufen werden mindestens zwei Lagertanks vorgehalten. Daneben wird ein Gaszwischenspeicher vorgehalten.

Die Belebungsanlage ist zweistraßig angelegt und besteht aus mehreren in Serie angeordneten Schlaufenreaktoren Die Belebung dient dem Abbau der Kohlenstoff-Fracht aus den Öl/Wasser-Gemischen, der Abwasserreinigung des anfallenden Prozeßwassers sowie der kombinierten Nitrifikation/Denitrifikation. Das vorgereinigte Abwasser wird nach Abtrennung der Biomasse über eine Separierungseinrichtung (Ultrafiltration, Schrägklärer. Zentrifuge o.a.) einer Pflanzenkläranlage zugeführt, wodurch das Abwasser Direkteinleiterqualität erreichen kann.

Die Fermentation ist zweistraßig angelegt und besteht aus jeweils einem mesophil und einem thermophil arbeitendem Fermenter. Die zugefügte Biomasse besteht aus Überschußschlamm aus der Belebungsstufe sowie direkt fermentierbare Flüssigkeiten. Durch den Einbau von Dreistoffdüsen in die Fermenter dienen diese gleichzeitig als Gasreinigung für das Pyrolysegas.

Der anfallende Faulschlamm wird über eine Sedimentationsstufe und eine Entwässerung (Zentrifuge, Hochdruck-Kammerfilterpresse o.a.) der Trocknung zugeführt. Der auf 95 % TS getrocknete Faulschlamm gelangt ebenfalls in den Misch- und Ausgleichsbehälter. Die zur Trocknung notwendige Energie wird dem Abgasstrom eines Blockheizkraftwerkes entnommen. Die anfallenden Brüdenkondensate gelangen zurück in die Fermentationsstufe.

Das gebildete Pyrolysegas wird in die Fermenter eingeblasen. Hierdurch wird die sonst notwendige und sehr aufwendige Gasreinigungsstufe bei Pyrolyse-Anlagen ersetzt. Das Pyrolysegas gelangt im Gemisch mit dem Biogas aus der Fermentation zur Gasaufbereitung. Der Pyrolysekoks gelangt über eine Transportvorrichtung zur Wirbelschichtfeuerung. Die Wirbelschichtfeuerung dient der indirekten Beheizung der Pyrolyse durch energetische Verwertung des anfallenden Pyrolysekokses. Bei unzureichendem Heizwert des Pyrolysekokses können die erforderlichen Temperaturen durch eine Stützfeuerung aufrecht erhalten werden.

Das erzeugte Prozeßgas setzt sich aus Bio- und Pyrolysegas zusammen. Vor der Verwendung als Treibstoff für die Kraft-Wärme-Kopplung wird das Gas von Siliziumverbindungen befreit, getrocknet und anschließend in den Gaszwischenspeicher eingeleitet. Der Gaszwischenspeicher dient gleichzeitig der Nivellierung der Gaszusammensetzung. Zur Energieerzeugung werden Blockheizkraftwerke neuester Bauart eingesetzt. Die überschüssige elektrische Energie wird über eine eigene Transformator-Station in das regionale Mittelspannungsnetz eingespeist. Überschüssige Wärmeenergie kann mittels eines eigenen Nahwärmenetzes an Betriebe in der Nachbarschaft abgegeben werden.

Die Steuerung der Biogas-Anlage erfolgt über eine speicherprogrammierbare Steuerung (SPS) mit angeschlossenem Zentralrechner mit Visualisierung. Die Bedienung der Anlage wird an Benutzerterminals durchgeführt. An der schematischen Darstellung der Anlage können alle Betriebsmittel zentral gesteuert und die Parameter der Regelkreise eingestellt werden. Hier laufen alle Betriebs- und Störmeldungen sowie Signale der Messwertaufnehmer auf und werden dargestellt. Der Zentralrechner der SPS übernimmt die Auswertung dieser Signale und steuert in deren Abhängigkeit die elektrischen Betriebsmittel.

Mittels Ankopplung über das Telefonnetz besteht die Möglichkeit, die Anlage über einen externen Rechner zu überwachen und zu steuern, ebenfalls mit visualisierter Darstellung. Kritische Betriebskennwerte und Störmeldungen werden per SMS an eine ständig besetzten Stelle gesendet. Somit ist eine ständige Überwachung empfindlicher und sicherheitsrelevanter Anlagenteile gewährleistet.

Der Bedarf an elektrischer Energie wird im normalen Betriebsfall durch Eigenversorgung gedeckt. Durch die zweistraßige Auslegung der Fermentation und der Belebung, den Einsatz von Zwillingspumpen sowie die parallele Versorgung über mindestens zwei BHKWs besteht Redundanz, so dass der Ausfall eines Teils der Anlage i.d.R. keine Unterbrechung des Prozesses bedingt.

Im Notfall übernimmt eine batteriegepufferte USV-Anlage die Versorgung des Prozessrechners bis die normale Energieversorgung in einem definierten Zustand weitergefahren wird. Für Anfahrbetrieb ist die Versorgung über Erdgas und elektrischen Strom aus dem öffentlichen Netz vorgesehen.

## Patentansprüche

1. Vorrichtung zum Verwerten von Flüssigabfällen, **dadurch gekennzeichnet, daß** die Vorrichtung eine Belebungsanlage und eine Fermentieranlage umfaßt, wobei eine Separiervorrichtung zum Trennen von fermentierbaren und nicht fermentierbaren Flüssigabfällen vorgesehen ist sowie Mittel zum Weiterleiten der fermentierbaren Flüssigabfälle zu der Fermentieranlage sowie Mittel zum Weiterleiten der nicht fermentierbaren Flüssigabfälle zu der Belebungsanlage vorgesehen sind, daß eine Sedimentationsstufe und eine Faulschlammentwässerungseinheit für den in der Fermentieranlage anfallenden Faulschlamm vorgesehen sind, daß sich an die Faulschlammentwässerungseinheit eine Faulschlammtrocknungseinheit anschließt und daß eine Pyrolyseeinheit zur Entgasung des getrockneten Faulschlamms aus der Faulschlammtrocknungseinheit vorgesehen ist.

2. Vorrichtung gemäß Anspruch 1, **dadurch gekennzeichnet, daß** die Belebungsanlage Mittel zum Abbau der Kohlenstoff-Fracht, Mittel zur Abwasserreinigung und kombinierten Denitrifikation umfaßt.

3. Vorrichtung gemäß Anspruch 2, **dadurch gekennzeichnet, daß** die Mittel zur Abwasserreinigung eine Pffanzenkläranlage umfassen.

4. Vorrichtung gemäß Anspruch 1, **dadurch gekennzeichnet, daß** die Fermentieranlage ein- oder mehrstraßig ausgebildet ist und einen oder mehrere mesophile und/oder thermophile Fermenter umfaßt.

5. Vorrichtung gemäß Anspruch 1, **dadurch gekennzeichnet, daß** Mittel zum Zuführen des Überschußschlamms aus der Belebungsanlage in die Fermentieranlage vorgesehen sind

6. Vorrichtung gemäß Anspruch 1, **dadurch gekennzeichnet, daß** die Faulschlammentwässerungseinheit als Zentrifuge ausgebildet ist.

7. Vorrichtung gemäß Anspruch 1, **dadurch gekennzeichnet, daß** Mittel zum Leiten des Pyrolysegases durch die Fermentierungsanlage vorgesehen sind.

8. Vorrichtung gemäß Anspruch 1, **dadurch gekennzeichnet, daß** Mittel zum Leiten von Biogas und des Pyrolysegases aus der Fermentierungsanlage zu einem Blockheizkraftwerk vorgesehen sind.

## Claims

1. Device for using liquid waste, **characterized in that** the device includes an activation station and a fermentation station, a separating device for separating fermentable and non-fermentable liquid waste being provided as well as means for transferring the fermentable liquid waste to the fermentation station and means for transferring the non-fermentable waste to the activation station, that a sedimentation stage and a digested-sludge dewatering unit are provided for the sludge produced in the fermentation station, that the digested-sludge dewatering unit is followed by a digested-sludge drying unit and that a pyrolysis unit is provided for degassing the dried digested sludge from the digested-sludge drying unit.

2. Device according to claim 1, **characterized in that** the activation station includes means for breaking down the carbon load and combined means for treating and denitrifying the wastewater.

3. Device according to claim 2, **characterized in that** the wastewater treatment means include a constructed-wetland wastewater treatment system.

4. Device according to claim 1, **characterized in that** the fermentation station has one or more lines and includes one or more mesophilic and/or thermophilic fermenters.

5. Device according to claim 1, **characterized in that** means are provided for supplying the excess sludge from the activation station to the fermentation station.

6. Device according to claim 1, **characterized in that** the digested-sludge dewatering unit is configured as a centrifuge.

7. Device according to claim 1, **characterized in that** means are provided for channeling the pyrolysis gas through the fermentation station.

8. Device according to claim 1, **characterized in that** means are provided for conveying biogas and the pyrolysis gases from the fermentation station to a combined heat and power plant.

## Revendications

1. Dispositif pour traiter des déchets liquides, **caractérisé en ce que** le dispositif comprend une installation d'activation des boues et une installation de fermentation, un dispositif pour séparer les déchets liquides pouvant être fermentés des déchets liquides ne pouvant pas être fermentés étant prévu ainsi que des moyens d'acheminement des déchets liquides pouvant être fermentés à l'installation de fermentation et des moyens d'acheminement des déchets liquides ne pouvant pas être fermentés à l'installation d'activation des boues, une étape de sédimentation et une unité de déshydratation des boues digérées étant prévues pour les boues digérées issues de l'installation de fermentation, une unité de séchage des boues digérées étant raccordée à l'unité de déshydratation des boues digérées et une unité de pyrolyse étant prévue pour le dégazage des boues digérées séchées issues de l'unité de séchage des boues digérées.

2. Dispositif selon la revendication 1, **caractérisé en ce que** l'installation d'activation des boues comprend des moyens de réduction de la charge de carbone, des moyens d'épuration et de dénitrification simultanée des eaux usées.

3. Dispositif selon la revendication 2, **caractérisé en ce que** les moyens d'épuration des eaux usées comprennent une station d'épuration des eaux usées végétale.

4. Dispositif selon la revendication 1, **caractérisé en ce que** l'installation de fermentation comprend une ou plusieurs voies et comprend un ou plusieurs fermenteurs mésophiles et/ou thermophiles.

5. Dispositif selon la revendication 1, **caractérisé en ce que** des moyens d'acheminement des boues excédentaires issues de l'installation d'activation des boues vers l'installation de fermentation sont prévus.

6. Dispositif selon la revendication 1, **caractérisé en ce que** l'unité de déshydratation des boues digérées est une centrifugeuse.

7. Dispositif selon la revendication 1, **caractérisé en ce que** des moyens de conduite des gaz de pyrolyse à travers l'installation de fermentation sont prévus.

8. Dispositif selon la revendication 1, **caractérisé en ce que** des moyens de conduite du gaz naturel et des gaz de pyrolyse de l'installation de fermentation vers une centrale thermique en montage-bloc sont prévus.
